# EUROPÄISCHE PATENTSCHRIFT

(11) **EP 1 637 147 B1**
(45) Veröffentlichungstag und Bekanntmachung des Hinweises auf die Patenterteilung: **10.12.2008**
(21) Anmeldenummer: 04022275.4
(22) Anmeldetag: 18.09.2004
(51) Int. Cl.: A61K 35/66, A61K 39/35, A61P 27/14, A61P 37/08, A61P 11/06

(54) **Stallstaub-Extrakt zum Schutz vor Allergien**
Stable dust extract for allergy protection
Extrait de poussières d'étable pour la protection des allergies

(43) Veröffentlichungstag der Anmeldung: 22.03.2006
(73) Patentinhaber: Protectimmun GmbH, 44879 Bochum (DE)
(72) Erfinder: Bufe, Albrecht, 20255 Hamburg (DE); Holst, Otto, 23843 Bad Oldesloe (DE); von Mutius, Erika, 82319 Leutstetten/Starnberg (DE); Braun-Fahrländer, Charlotte, 4052 Basel (CH); Nowak, Dennis, 82152 Krailling (DE); Riedler, Josef, 5500 Bischofshofen (AT)
(74) Vertreter: Polypatent

(56) Entgegenhaltungen:
- WO-A-01/49319
- WO-A-94/06821
- DE-A1- 4 422 859
- US-A- 4 234 569
- RIEDLER J ET AL: "Austrian children living on a farm have less hay fever, asthma and allergic sensitization" CLINICAL AND EXPERIMENTAL ALLERGY, Bd. 30, Nr. 2, Februar 2000 (2000-02), Seiten 194-200, XP002318936 ISSN: 0954-7894

## Beschreibung

Die vorliegende Erfindung betrifft eine Zusammensetzung, welche die gesamte Allergie protektive Potenz, Aktivität und Wirkung eines natürlichen Stallstaubs enthält, und ein Verfahren zur Herstellung dieser Zusammensetzung.

Als Allergien bezeichnet man überschießende Reaktionen des Körpers, insbesondere des Immunsystems, auf nicht schädliche fremde Substanzen. Diese Reaktionen laufen genauso wie die normale Immunantwort gegen einen Krankheitserreger ab. Es werden mehrere Typen der allergischen Immunantwort unterschieden. Allergische Reaktionen vom Typ 1, zu denen bronchiales Asthma, Atopische Dermatitis, Urtikaria (Nesselsucht), Heuschnupfen sowie Nahrungsmittelallergien gehören, sind am weitesten verbreitet. Obgleich diese Krankheiten unterschiedliche körperliche Symptome aufweisen, liegen Ihnen ähnliche immunologische Mechanismen zugrunde.

Zahlreiche Studien belegen, dass allergische Erkrankungen zunehmen. Die Ursachen der Entstehung und Entwicklung der Allergien sind bisher unklar. Bestimmte bisher unbekannte Erbfaktoren und Umweltbedingungen wie Allergenexposition, Leben in der Stadt, Anzahl der Geschwister und manche Infektionen tragen wahrscheinlich zur Entstehung bei.

Allergien vom Typ I werden durch Antikörper der Gruppe E vermittelt, deren Entstehung von T-Helferlymphozyten der Gruppe II abhängig ist. Diese Th2-Zellen kommen bei Allergien im Verhältnis zu den Th1 Zellen häufiger vor. Kinder werden zunächst mit einer Th2 dominierten Immunantwort geboren, die sich im ersten Lebensjahr wohl unter dem Einfluß der mikrobiellen Belastung zu einer Th1 dominierten Antwort (nicht allergischen Immunantwort) verschiebt. Es wird angenommen, dass häufige Infektionen mit einer geringeren Th2 Antwort und damit weniger Allergien korrelieren. Eine niedrige mikrobielle Belastung könnte also zum verstärkten Auftreten von Allergien führen (Hygienehypothese). Eine Reihe von epidemiologischen Studien in Europa haben gezeigt, dass Kinder, die auf Bauernhöfen aufwachsen sehr viel weniger allergische Erkrankungen aufweisen als Kinder, die unter nicht traditionellen Lebensbedingungen groß werden (Bauerneffekt). Es ist heute klar, dass dieser Effekt mit dem häufigen Stallkontakt der Kinder auf den Bauernhöfen zusammenhängt, da Allergieprotektion am deutlichsten bei Kindern zu finden war, die sich im ersten Lebensjahr häufig dort aufhielten. Dieser Schutzeffekt geht mit sehr hoher Wahrscheinlichkeit vom Kontakt mit Stallstaub (z. B. über Inhalation) aus. Das hängt damit zusammen, dass der Stallstaub unter anderem auch immunstimulatorisch wirksame Substanzen aus Bakterien, Viren, Pilzen, Pflanzen und Tieren enthält, die vermutlich den mit der Hygienehypothese zusammenhängenden Bauerneffekt ausmachen. Welche Substanzen im Stallstaub den Schutzeffekt letztlich auslösen, ist jedoch bis heute unbekannt.

Die Möglichkeiten zur Prävention und Therapie allergischer Erkrankungen sind begrenzt. Die Symptome lassen sich zwar relativ gut durch zahlreiche Medikamente lindern, die immuntherapeutischen Behandlungen wie die Desensibilisierung sind aber unterschiedlich erfolgreich. Vor allem bei dem häufig auftretenden bronchialen Asthma ist diese Therapie eher unwirksam. Auch die Allergenvermeidung als präventive Maßnahme trägt nicht sicher zur Senkung der Allergiehäufigkeit bei. Insgesamt gibt es keine Möglichkeiten, dass Risiko für Allergieentstehung zu senken.

Die WO/0149319 offenbart, dass Stallstaub mittels eines Luftfilters direkt in eine wässrige Lösung, z.B. in eine physiologische Salzlösung, gesammelt und dann direkt zur Vorbeugung von Allergien bei Kindern verwendet werden kann.

Die im Staub enthaltenen Mikroorganismen können auch aufgeschlossen oder aufgereinigt werden. Im Falle eines Aufschlusses sind die isolierten Zelltrümmer, Membranstücke, Organellen und Proteine = Antigene von Interesse. Die Aufschlussfragmente können weiter gereinigt werden, dh Nukleinsäuren und andere nicht erwünschte Bestandteile können entfernt werden durch Zentrifugation, chromatographische Verfahren, Gelfiltration und/oder Aussalzung.

Im Gegensatz dazu wird erfindungsgemäss trockener Stallstaub gesammelt und der flüssige Überstand der wässrigen Extraktion nach einer Sedimentation verwendet.

Die WO 96/00579 beschreibt ein Herstellungsverfahren zum Herstellen einer Suspension und eines Extraktes von Mykobakterien in wässriger Lösung, bei dem der Extrakt wenigstens 20 Minuten auf 121°C erhitzt wird. Diese Suspension oder dieser Extrakt kann für die unspezifische Immunmodulation eingesetzt werden.

Die Aufgabe der vorliegenden Erfindung war es ein Verfahren zur Herstellung eines Mittels und ein Mittel zur Vorbeugung und Behandlung von allergischen Erkrankungen bereitzustellen.

Diese Aufgabe wird gelöst durch ein Verfahren gemäß Anspruch 1.

Der Extrakt wird aus Stallstaub, bevorzugt von Bauernhöfen, hergestellt.

Der Staub kann mit Hilfe jeder Art von Aufnahmesystem gesammelt werden, also durch Zusammenkehren, Aufsaugen, Abwischen und ähnlichem, ohne dass die Erfindung hierdurch beschränkt würde.

Nach dem Sammeln kann der Staub homogenisiert werden. Hierfür ist jede Art von Technik möglich, die zu einer gleichmäßigen Homogenisierung des Staubes führt, insbesondere die Verklebungen und Verklumpungen des Staubes aufhebt. Hierfür sind Verfahren wie das Reiben, Zerschlagen oder Zerstampfen, Rühren oder Einbringen in einen Mixer geeignet, ohne hierauf beschränkt zu sein. Auch ein Aufschließen des Staubes kann Bestandteil des erfindungsgemäßen Verfahrens sein. Ein Aufschließen der in dem Staub vorliegenden Bestandteile wie Zellen, Mikroorganismen, insbesondere deren Sporen und Ähnliches kann z.B. durch Zerreiben, Zerquetschen und ähnliche Verfahren erfolgen.

Die Extraktion des Staubes wird erfindungsgemäß bevorzugt mit Hilfe von Wasser oder einer wässrigen Lösung durchgeführt, besonders bevorzugt ist eine physiologische wässrige Kochsalzlösung oder ein wässrigen Puffer. Ein bevorzugter wässriger Puffer enthält Natriumsalze oder ähnliche ein- oder zweiwertige Salze wie Na₂SO₄, KCl, LiCl, MgCl₂ und CaCl₂. Ganz besonders bevorzugt ist es die Extraktion des Staubes mit einer physiologischen Kochsalzlösung (0,9% NaCl) durchzuführen.

Ein wichtiger Aspekt des erfindungsgemäßen Verfahrens ist, dass während des gesamten Aufbereitungsverfahrens der Herstellung des Extraktes keine externe Wärmezufuhr erfolgt. Dies bedeutet, dass weder die eingesetzten Lösungsmittel (zum Zeitpunkt des Einsatzes) erhitzt werden, noch während des Herstellungsverfahrens eine Wärmezufuhr erfolgt. Vielmehr wird der Extrakt bei Umgebungstemperatur oder darunter hergestellt. Dies bedeutet, dass alle verwendeten Lösungsmittel Umgebungstemperatur (je nach Jahreszeit und Herstellungsort zwischen ca. 10°C und 40°C) haben, oder gekühlt sein können, nicht aber in heißem Zustand verwendet werden, oder der Extrakt erhitzt wird.

Nach Aufnahme des Staubes in der wässrigen Lösung oder Wasser wird diese Suspension entweder stehen gelassen oder gerührt, so dass die wasserlöslichen oder durch Wasser von den Stäuben entfernbaren Substanzen in die wässrige Phase übergehen können. Anschließend werden die in der Lösung als feste Bestandteile verbleibenden Staubanteile durch einen Sedimentationsschritt entfernt. Der Sedimentationsschritt ist für das erfindungsgemäße Verfahren nicht limitierend, es kann jede dem Fachmann bekannte Art der Sedimentation angewendet werden. Auch das Stehenlassen der Suspension, so dass das Sedimentieren der festen Bestandteile durch die Erdanziehung erfolgt kann als im Sinne der Erfindung betrachtet werden. Eine bevorzugte Art des Abtrennens der festen Bestandteile ist das Zentrifugieren, wonach der Überstand von dem Sediment abgenommen wird. Der Überstand, aus dem die festen Bestandteile wie Zellen, Mikroorganismen oder Sporen aus den Stäuben durch Sedimentieren abgetrennt wurden, bildet den erfindungsgemäßen Extrakt.

In einer bevorzugten Ausführungsform der Erfindung kann der Extrakt optional noch einem Dialyseschritt unterzogen werden. Die Dialyse kann gegen dieselbe Art von Lösungsmitteln erfolgen, wie sie bereits für die Aufnahme des Staubes eingesetzt wurden. Bevorzugt erfolgt die Dialyse gegen aqua dest. Eine bevorzugte Ausschlussgrenze bei der Dialyse stellt eine Grenze von ca. 3000 Dalton dar. Somit werden kleinere Moleküle aus dem Extrakt entfernt.

Ein nach diesem Verfahren hergestellter Extrakt zeichnet sich qualitativ dadurch aus, dass er bei gleichen Einsatzmengen von Staub und gleichen Einsatzmengen an Lösungsmitteln einen deutlich höheren Proteinanteil enthält als Extrakte, die nach herkömmlichen Extraktionsverfahren hergestellt werden, außerdem ist die Proteinverteilung (das Proteinmuster auf einem SDS Gel) von der anderer Extrakte zu unterscheiden. Der Extrakt zeichnet sich weiterhin dadurch aus, dass er Saponine zu einem deutlichen Anteil enthält.

Der erfindungsgemäße Extrakt kann für die Vorbeugung und Behandlung von allergischen Erkrankungen verwendet werden, insbesondere kann er als Medikament oder für die Herstellung eines Medikaments für die Vorbeugung und Behandlung von allergisch bedingten Erkrankungen wie Asthma bronchiale, Heuschnupfen, atopische Dermatitis, Nahrungsmittelallergien, Urtikaria und alle Arten von Kontaktallergien verwendet werden. Die Wirksamkeit des erfindungsgemäßen Extraktes übersteigt deutlich die von Extrakten, die nach herkömmlichen Verfahren hergestellt werden, wie in den Beispielen gezeigt wird.

### Abbildungen:

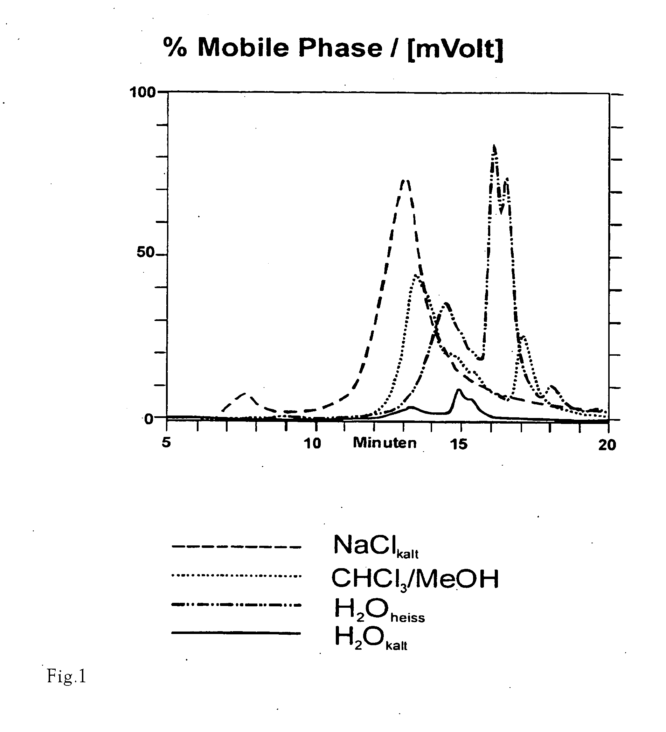

**Abb. 1** zeigt das Trennungsprofil der Auftrennung der erfindungsgemäßen Zusammensetzung und dreier Vergleichsextrakte mittels HPLC.

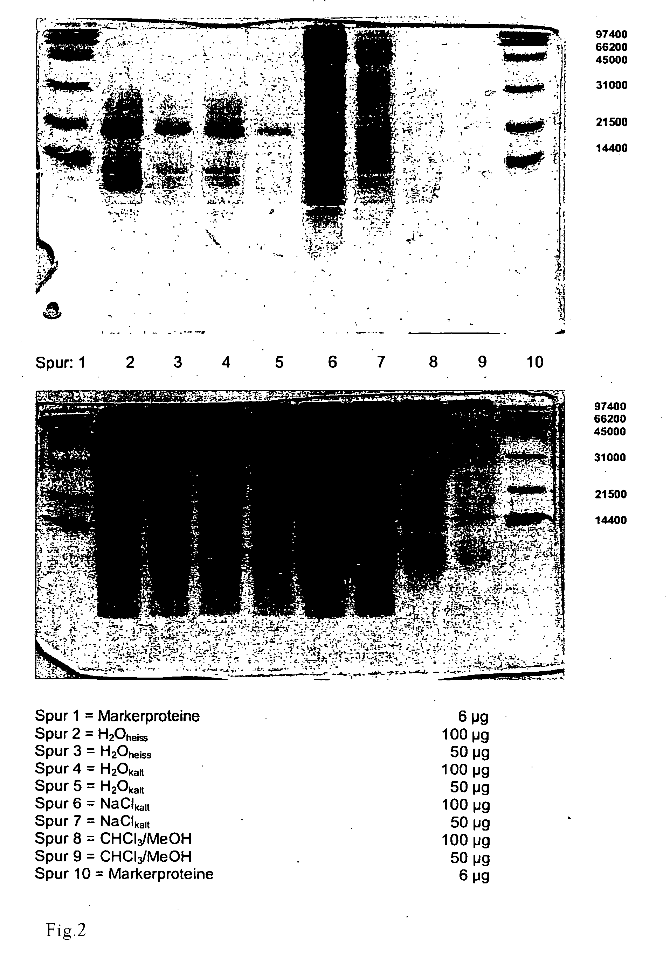

**Abb. 2** zeigt die Auftrennung der erfindungsgemäßen Zusammensetzung und dreier Vergleichsextrakte mittels SDS-PAGE. Das obere Gel ist Coomassi blue gefärbt, das untere zeigt eine Silberfärbung derselben Auftrennung.

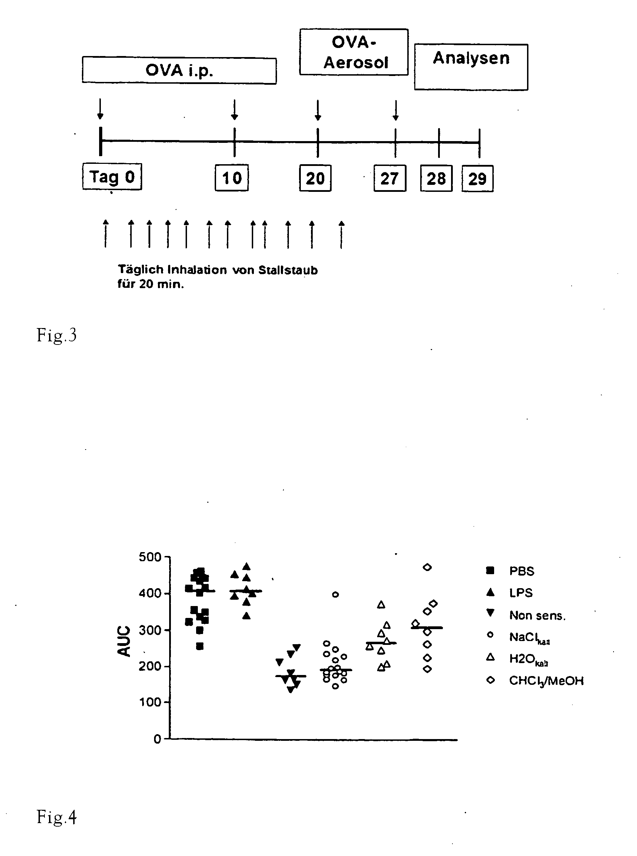

**Abb. 3** zeigt eine schematische Zusammenfassung des Asthma- und Sensibilisierungsmodell in der Balb/c Labormaus. Die Mäuse erhalten 2x im Abstand von 14 Tagen das Allergen (Ovalbumin Grad VII) intraperitonial, anschließend 2x als Aeorosol. Parallel inhalieren die Mäuse in einer abgeschlossenen Kammer die erfindungsmäßige Zusammensetzung für 20 Minuten täglich über 14 Tage. Am 39. Tag wird die Atemfunktion der Lunge mit dem Bodyplethysmographen für die Maus gemessen, am **41**. Tag folgen die übrigen serologischen und zellbiologischen Analysen.
**Abb. 4** zeigt die Ergebnisse der Untersuchungen von Asthmasymptomen bei Mäusen. AUC steht für "area under the curve" und beschreibt die Fläche unter der Penh-Kurve nach Stimulation der Mäuse mit 6, 12, 24 und 48 mg/ml Metacolin. Es werden Mäuse verglichen, die PBS als negative Kontrolle (***PBS***), LPS in Stallstaub äquivalenter Dosis als negativ Kontrolle (***LPS***), die erfindungsgemäße Zusammensetzung (***NaCl**ₖₐₗₜ*), ein Kaltwasserextrakt zum Vergleich (***H₂O**ₖₐₗₜ*), die Methanolphase des Bligh&Dyer Extrakts (***CHCl₃*/*MeOH***) und PBS ohne Sensibilisierung (***non sens.***) inhaliert haben.

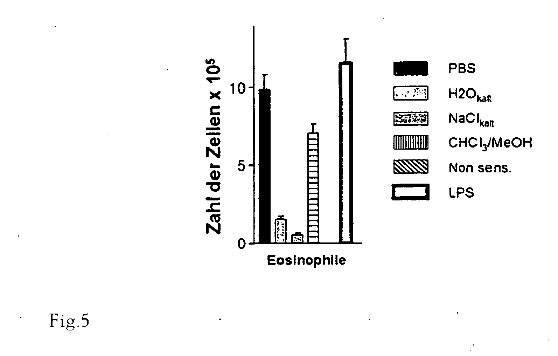

**Abb. 5** zeigt die Modulation der Eosinophilen Zellen in der bronchoalveolären Lavage (BAL). Es wurden Mäuse verglichen, die PBS als negative Kontrolle (***PBS***), LPS in Stallstaub äquivalenter Dosis als negativ Kontrolle (***LPS***), die erfindungsgemäße Zusammensetzung (***NaCl**ₖₐₗₜ*), ein Kaltwasserextrakt zum Vergleich (***H**₂**O**ₖₐₗₜ*), die Methanolphase des Bligh&Dyer Extrakts (***CHCl₃*/*MeOH***) und PBS ohne Sensibilisierung (***non sens.***) inhaliert haben.

### Beispiele:

### Beispiel 1: Stall- und Scheunenstaubsammlung :

Die Sedimentstaubsammlung erfolgt bevorzugt aus Kuh- und Kälberställen und Scheunenbereichen traditionell geführter Alpenbauernhöfe. Diese sind definiert über ihre Lage in den nördlichen Alpentälern in der Schweiz, Österreichs und Deutschlands und ihre besondere Architektur, d.h. der räumlichen Nähe des Stall-und des Wohnbereichs. Die Probenahme des Sedimentstaubs erfolgt von den Scheunentorsimsen, den Fenstersimsen, erhöhten Ablageflächen, Kuhtrainer-Gestängen, Tablaren, Schemeln, erhöhte Ablageflächen und herumliegenden Werkzeugen. Die Höhe der Sammelflächen sollte zwischen 0.5m und 1.5m Höhe über Boden betragen. Es darf kein feuchter oder sogar nasser Staub gesammelt werden. Die Oberflächen werden mit dem Metallspatel abgekratzt und durch das Haushaltssieb (ALK-Scherax Filterhalteraufsatz [Vorabscheidersieb] für Staubsauger) in den Staubsammelbehälter für Stallstaub gesiebt.

Die gesammelten Proben werden alle im geschlossenen Sammelbehälter trocken bei Raumtemperatur (maximal 22°C) und vor direkter Sonneneinstrahlung geschützt aufbewahrt.

### Beispiel 2: Extraktion:

Der Stallstaub wird mit einem Spatel und anschließend im Mörser homogenisiert. Anschließend wird der so homogenisierte Staub in 0.9% wässriger NaCl-Lösung suspendiert (bei 22°C), mittels Glasperlen bei ca. 4°C aufgeschlossen und bei 22°C für weitere 6 Std. vorsichtig gerührt. Anschließend wird zentrifugiert, der Überstand wird abgenommen, gegen Wasser dialysiert und lyophilisiert. Die Ausbeute des NaClₖₐₗₜ Extrakts (erfindungsgemäße Zusammensetzung) liegt bei 9 Gew.-% des eingesetzten Stallstaubs. Zum Vergleich wurden Extrakte aus Stallstaub mit H₂Oₖₐₗₜ (16.8 Gew.-%), H₂Oₕₑᵢₛₛ (20.2 Gew.-%) und CHCl₃/MeOH (9 Gew.-%) hergestellt.

### Beispiel 3:Biochemische Charakterisierung

Der erfindungsgemäße Zusammensetzung wird im Vergleich zu den drei anderen Extrakten einer fingerprint-Analyse mittels HPLC unterworfen und durch SDS-Polyacrylamid-Gelelektrophorese (SDS-PAGE) auf das Vorkommen von Proteinen (Coomassie-Färbung, Silberfärbung) und Lipoglycanen (Silberfärbung) charakterisiert. Weiterhin werden Zucker, Protein (Gesamtgehalt) und Fettsäuren quantifiziert. Dazu werden standardisierte Routinemethoden angewandt.

### Charakterisierung der Extrakte durch HPLC und SDS-PAGE:

Die Extrakte werden zur Charakterisierung mittels HPLC über eine Zorbax Bioseries GF-250 Säule aufgetrennt (0.2 M Na-Phosphat-Puffer, pH 7.0, isokratisch, Auftrennung von Proteinen nach Größe, Detektion bei 280 nm). Im Vergleich zu den anderen Staubextrakten ist für die erfindungsgemäße Zusammensetzung bei 280 nm ein charakteristisches Trennprofil erkennbar, welches in Abbildung 1 gezeigt ist.

Die Auftrennung der Extrakte in der SDS-PAGE (Abb. 2) zeigt, dass sich die erfindungsgemäße Zusammensetzung sowohl qualitativ als auch quantitativ von den anderen Extrakten unterscheidet. Dies betrifft sowohl Proteine (Coomassiefärbung, oberes Bild: MGW 30000 - 100000 Da) als auch Lipoglycane (Silberfärbung: breit schmierende Substanz(en) im Bereich MGW 20000 und darunter). Dazu zeigt die Silberfärbung weitere distinkte Banden, die nicht oder nur sehr schwach durch Coomassie angefärbt werden (MGW ca. 26 kDa, ca. 14 kDa, ca. 12 kDa und 2 Banden darunter). Hierbei könnte es sich um Proteine und/oder Glycoproteine handeln, die nur sehr schlecht mit Coomassie anfärben (stark saure Proteine) und/oder nur in geringen Mengen vorliegen (die Silberfärbung ist zwischen 10 und 40 mal empfindlicher als die Coomassiefärbung). Insbesondere enthält der erfindungsgemäße Extrakt deutlich mehr Proteine hoher Molekulargewichte als die Vergleichsextrakte, wobei in allen Extrakten ein Protein mit ca. 20 kDa besonders präsent ist.

Der Proteingehalt der erfindungsgemäßen Zusammensetzung liegt mit ca. 140 µg/mg um das Zweifache höher als die Proteinmengen der anderen Extrakte, wenn von gleichen Ausgangsmengen an Stallstaub ausgegangen und dieselben Volumina im Verfahren verwendet wurden.

Die Analyse der Zuckerzusammensetzung zeigte, dass in allen Extrakten keine Uronsäuren und Heptosen zu finden sind und alle Extrakte Glucosamin und Galactosamin enthalten. Hinsichtlich der Neutralzucker findet man in der erfindungsgemäßen Zusammensetzung 34,6 nmol/mg Rhamnose, 20,9 nmol/mg Ribose, 42,0 nmol/mg Arabinose, 25,6 nmol/mg Xylose, 70,9 nmol/mg Mannose, 129,7 nmol/mg Glucose, und 124,4 nmol/mg Galactose.

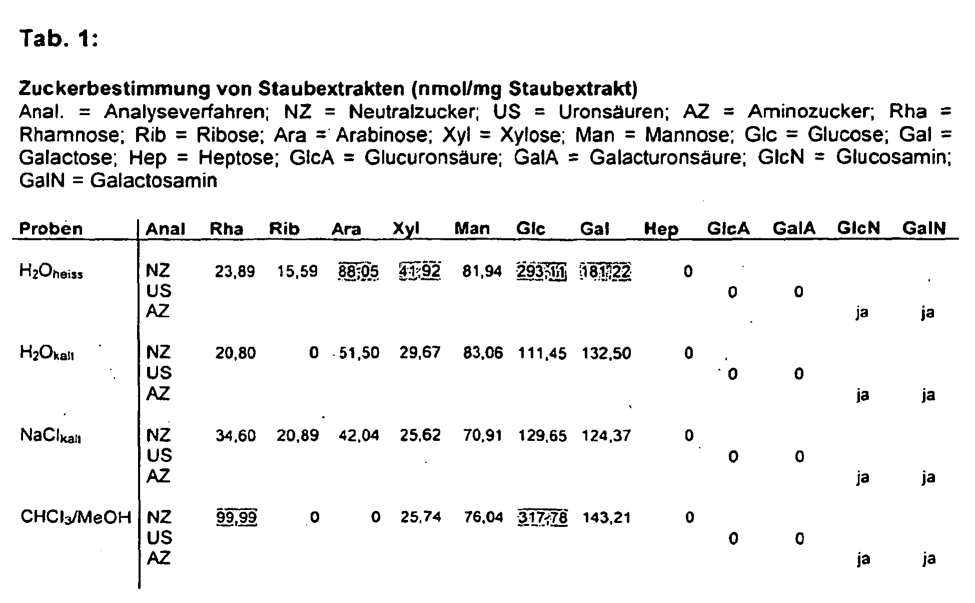

Wie aus Tabelle 1 ersichtlich ist, besitzt H₂Oₕₑᵢₛₛ deutliche höhere Mengen an Arabinose, Xylose, Glucose und Galactose, und der CHCl₃/MeOH Extrakt höhere Mengen an Rhamnose und Glucose. H₂Oₖₐₗₜ unterscheidet sich in seiner Neutralzuckerzusammensetzung nicht wesentlich von der erfindungsgemäßen Zusammensetzung. Diese Befunde deuten darauf hin, dass die Temperatur bei der Extraktion für molekulare Unterschiede wesentlich mit verantwortlich ist.

Die Analyse der Fettsäuren ergibt für die erfindungsgemäße Zusammensetzung einen Gehalt an C12:0 von 5.9 nmol/mg, C14:0 von 30.7 nmol/mg, 2-OH-C14:0 von 4.3 nmol/mg, 3-OH-C14:0 von 3.2 nmol/mg, C16:0 von 13.9 nmol/mg, C18:0 von 5.6 nmol/mg und C18:1 von 4.7 nmol/mg. In der Summe bedeutet das 68.2 nmol/mg Fettsäuren.

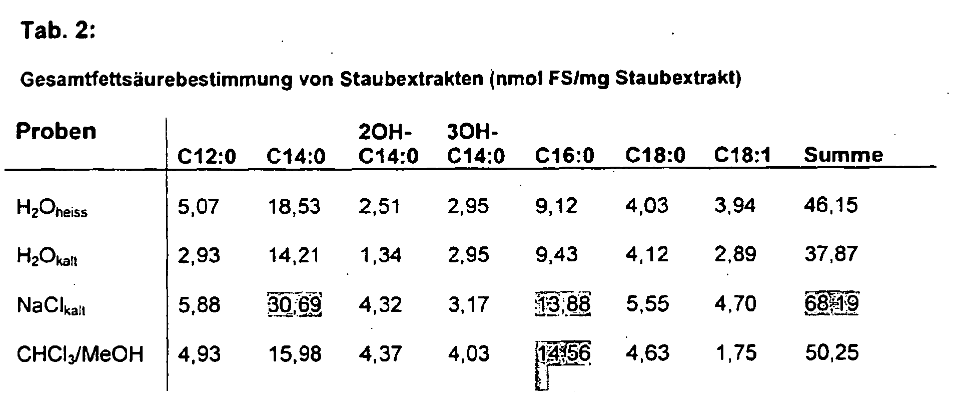

Wie Tabelle 2 zeigt, weist die erfindungsgemäße Zusammensetzung einen deutlich höheren Gehalt an C14:0 und C16:0 als die anderen beiden wässrigen Extrakte auf. Diese Ergebnisse zeigen, dass neben der Temperatur auch der Zusatz von NaCl zur Extraktion von unterschiedlichen Molekülen aus Stallstaub führt.

### Beispiel 4: Effektivität in vivo

Die vollständige protektive Potenz der erfindungsgemäßen Zusammensetzung wird durch immunologische und zellbiologische Funktionsuntersuchungen im standardisierten Tiermodell der Maus *in vivo* bestimmt.

Im folgenden werden zunächst die Methoden zur Sensibilisierung und Induktion von allergischem Asthma bei Mäusen mit Ovalbumin, der Behandlung mit StallstaubExtrakten und der Analyse der allergischen und entzündlichen Reaktionen beschrieben.

***Sensibilisierung und Induktion von allergischem Asthma bei Mäusen mit Ovalbumin (OVA)**-* Zur Sensibilisierung von Mäusen gegen das Modell-Allergen Ovalbumin wird Aluminiumhydroxid (Pierce) als Adjuvans verwendet. Von diesem Adjuvans ist bekannt, dass es Immunantworten auslöst, die durch die Produktion von Cytokinen wie IL-4, IL-5 und IL-13 charakterisiert sind und durch die Produktion von Antikörpern des Isotyps IgE und IgG1 (entsprechender Isotyp beim Menschen ist IgG4).
Die Sensibilisierung von sieben Wochen alten Mäusen des Inzuchtstammes Balb/c erfolgt durch intraperitoneale Injektion eines Gemisches von 20µg Ovalbumin (Grade V der Firma Sigma) und 2.2 mg Aluminiumhydroxid in einem Gesamtvolumen von 200µl Puffer (PBS). Insgesamt erfolgt die Injektion zweimal, nämlich am Tag 0 und am Tag 14.
Um anschließend eine allergische Reaktion in der Lunge der Mäuse hervorzurufen, also das akute allergische Asthma auszulösen, werden die Mäuse am Tag 28 und Tag 38 jeweils 20 Minuten lang mit Ovalbumin-Aerosol behandelt. Hierzu werden die Mäuse in eine Plexiglas-Kammer mit 11 Litern Volumen gesetzt. An die Kammer wird ein Inhalator der Firma Pari angeschlossen, der zuvor mit 1 %iger Ovalbuminlösung befüllt wurde, und mit einem Pari-Boy-Turbo Aerosol Generator betrieben wird. Nach der zweiten Applikation von OVA-Aerosol kann bei den Mäusen einige Tage lang Symptome des akuten allergischen Asthmas diagnostiziert werden. Zu den Symptomen gehören:
1) die verstärkte Reaktion gegen Methacholin, die bronchiale Hyperreaktivität
2) die verstärkte Infiltration von eosinophilen Granulozyten und Lymphozyten in das Atemwegslumen.
3) die Produktion von Ovalbumin spezifischen Antikörpern der Isotypen IgE und IgG1
4) die Produktion von Interleukin-5 nach in vitro Restimulierung von Lymphozyten mit Ovalbumin

Die Methoden zur Messung der einzelnen Parameter werden im folgenden näher erläutert.

***Messung der bronchialen Hyperreaktivität** -* Ein entscheidender diagnostischer Parameter, der beim Menschen gemessen wird, um eine allergisch asthmatische Erkrankung sicher zu diagnostizieren, ist die bronchiale Hyperreaktivität. Hierbei handelt es sich um eine Überempfindlichkeit der glatten Muskulatur der Bronchien gegen unspezifische Reizungen. Zur Diagnose beim Menschen werden geringe Konzentrationen an Histamin appliziert und anschließend der Atemwegswiderstand in einem Plethysmographen gemessen. Asthmatische Personen zeigen eine von der Histaminkonzentration abhängige stärkere Bronchokonstriktion und damit einen höheren Atemwegswiderstand als gesunde Personen.
Ganz ähnlich erfolgt die Messung bei Mäusen. Zur Messung werden die Mäuse in einen Plethysmographen der Firma Buxco in die dafür vorgesehenen Kammern gesetzt. Durch die Kammern fließt ein kontinuierlicher Luftstrom und ein hochempfindlicher Druckabnehmer registriert kurzfristige Druckänderungen in der Kammer. Die Einatmung der Mäuse erzeugt einen Unterdruck und die Ausatmung einen Überdruck in der Kammer. Diese Druckänderung werden in Abhängigkeit von der Zeit kontinuierlich aufgezeichnet und von der Software aus den gemessenen Werten für jeden Atemzyklus der sogenannte Penh-Wert berechnet. Die Formel mittels der der Penh-Wert berechnet wird, geht aus Abbildung 5 hervor. Dieser dimensionslose Wert korreliert mit dem Atemwegswiderstand. Bei hyperreaktiven Mäusen erhöht sich der Atemwegswiderstand abhängig von der Methacholin-Konzentration (bei Mäusen nimmt man anstelle von Histamin, Methacholin zur Provokation, da die Bronchien von Mäusen nicht auf Histamin reagieren), was sich durch die Erhöhung des Penh-Wertes ausdrückt.
Die Messung der bronchialen Hyperreaktivität erfolgt 24 Stunden nach der letzten OVA-Aerosol Exposition. Für die Messung der bronchialen Hyperreaktivität werden die Mäuse nach einer Gewöhnungszeit von 10 Minuten im Plethysmographen 7 Minuten lang mit PBS-Aerosol in Kontakt gebracht und dabei der Penh-Wert aufgezeichnet. Anschließend erfolgt die Exposition jeweils 7 Minuten lang mit aufsteigenden Konzentrationen an Methacholin: 6, 12, 25, 50mg/ml. Während jeder Exposition wird der Penh-Wert kontinuierlich gemessen und über Perioden von 30 Sekunden gemittelt. Die Periode in der für eine bestimmte Methacholin-Konzentration der maximale Penh-Wert erreicht wird, wird dann gegen die Methacholin-Konzentration aufgetragen. Hieraus ergibt sich eine Dosis-Wirkungs-Kurve, deren Fläche unter der Kurve umso größer ist, je stärker die Mäuse auf Methacholin reagieren. Die Fläche unter der Kurve korreliert also mit der Hyperreagibilität der Tiere und wird zur statistischen Auswertung verwendet.

***Untersuchung der zellulären Zusammensetzung der Broncho-Alveolären-Lavage (BAL)*** - Bei gesunden Probanden bzw. unbehandelten Mäusen können nur sehr wenige bis gar keine eosinophile Granulozyten im Lungengewebe und im Lumen der Atemwege beobachtet werden. Die Leukozyten, die in gesunden Atemwegen vorgefunden werden, sind hauptsächlich Makrophagen. Im Zusammenhang mit allergischem Asthma kommt es aus noch nicht ganz geklärten Ursachen zur massiven Infiltration der Atemwege durch eosinophile Granulozyten und im geringeren Maße durch Lymphozyten.
Um die Zusammensetzung des Leukozyten-Infiltrats in den Atemwegen von Mäusen zu untersuchen, werden diese drei Tage nach der letzten OVA-Aerosol Exposition getötet. Die Trachea wird freigelegt und eine 24G Venen-Verweilkanüle in die Trachea inseriert. Durch diese wird das Lumen der Atemwege zweimal mit jeweils 1ml isotonischem Puffer gewaschen (Broncho-Alveoläre-Lavage). Die Gesamtzahl der Zellen, die auf diese Weise aus der Lunge herausgespült werden, wird mittels Neubauer Zählkammer mikroskopisch ausgezählt. Anschließend werden die Leukozyten der BAL mit einer Zyto-Zentrifuge der Firma Shandon auf Objektträger zentrifugiert. Die Färbung der Zellen erfolgt dann mit dem HAEME-Schnellfärbungs Kit der Firma Labor+Technik Eberhard Lehmann, wie in der Produktbeilage beschrieben. Nach erfolgter Färbung werden 300 Zellen auf dem Objektträger mikroskopisch ausgezählt und nach den gängigen Kriterien in die unterschiedlichen Leukozyten-Subpopulationen eingeteilt. Anschließend kann die prozentuale Zusammensetzung der BAL berechnet werden.

***Messung von OVA-spezifischen Antikörpern des Isotyps IgE und IgG1* -** Die Sensibilisierung gegen ein Allergen kann durch die Bestimmung von Allergen-spezifischen Antikörpern der Klassen IgG1 und IgE gemessen werden. Die Quantifizierung der Allergen-spezifischen Antikörper im Serum von Mäusen erfolgt mittels indirektem ELISA. Hierzu wird den Mäusen zwei Tagen nach der letzten Applikation von OVA-Aerosol Blut aus der Schwanzvene entnommen. Nach dem Gerinnen des Bluts wird das Serum durch Zentrifugation gewonnen und im ELISA eingesetzt:

### Kurzanleitung für ELISA:

### lgE (OVA-spez.)-ELISA:

- Platten beschichten mit 5µg/ml OVA in Beschichtungspuffer, je 100µl pro Depot (über Nacht)
- 3x waschen, jeweils 250µl pro Depot
- Nachbeschichtung mit 1% BSA in Waschpuffer, 250µl pro Depot, 1 Stunde
- 3x waschen
- Serumverdünnungen in Waschpuffer auftragen 1/3 1/10 und 1/30, 100µl pro Depot, 1 Stunde
- 3x waschen
- biotinylierten anti-IgE-Antikörper (Pharmingen, R35-72) 1/100 in Waschpuffer verdünnt auftragen, 100µl pro Depot, 1 Stunde
- 3x waschen
- Extravidin-POD (Sigma) 1/1000 in Waschpuffer verdünnt auftragen, 100µl pro Depot, 1 Stunde
- 3x waschen
- TMB-Substratlösung auftragen, 100µl pro Depot, 30min
- Reaktion abstoppen mit 50µl 1 M Schwefelsäure pro Depot, Platte kann bei einer Wellenlänge von 450nm gemessen werden

### IgG1 (OVA-spez.)-ELISA:

- Platten beschichten mit 5µg/ml OVA in Beschichtungspuffer, je 100µl pro Depot (über Nacht)
- 3x waschen, jeweils 250µl pro Depot
- Nachbeschichtung mit 5% MMP in Waschpuffer, 250µl pro Depot, 1 Stunde
- 3x waschen
- Serumverdünnungen in Verdünnungspuffer auftragen 1/100 1/300 1/1000 und 1/3000, 100µl pro Depot, 1 Stunde
- 3x waschen
- AKP-konjugierten anti-lgG1-Antikörper (Pharmingen, X56) 1/1000 in Verdünnungspuffer verdünnt auftragen, 100µl pro Depot, 1 Stunde
- 3x waschen
- pNPP-Substratlösung auftragen, 100µl pro Depot, 30min
- Reaktion abstoppen mit 50µl 1M Natronlauge pro Depot, Platte kann bei einer Wellenlänge von 405nm gemessen werden

Auf jeder ELISA-Platte wird eine Verdünnungsreihe eines Standard-Serums mitgeführt. In diesem Standard-Serum befindet sich eine bekannte Konzentration an OVA-spezifischen Antikörpern des Isotyps IgG1 und IgE. Durch den Vergleich von Messwerten aus Mausserum mit unbekannter OVA-spez. IgG1- und IgE-Konzentration mit der Messreihe des bekannten Mausserums, kann die Konzentration in der unbekannten Probe berechnet werden.

### Behandlung von Mäusen mit Stallstaubextrakten während der Sensibilisierung:

Zur Behandlung von Mäusen mit den unterschiedlichen Stallstaubextrakten werden diese in eine Plexiglaskammer mit 11 Litern Volumen gesetzt. Die Kammer wird, wie oben im Absatz *"Sensibilisierung und Induktion von allergischem Asthma bei Mäusen mit Ovalbumin (OVA)"* beschrieben, an einen Pari-Boy Aerosol-Generator angeschlossen, wobei der Inhalator zuvor mit einer 1%igen Lösung der Stallstaubextrakte gefüllt wurde. Anschließend werden die Mäuse 20 Minuten lang mit dem Stallstaubextrakt-Aerosol behandelt. Die Behandlungen erfolgen insgesamt 14x und zwar zu den in Abbildung 3 spezifizierten Zeitpunkten während der Sensibilisierungen.

### Beispiel 5: Ergebnisse der in vivo Versuche

***Asthmasymptome** -* Der am weitesten gehende Versuch zum Nachweis der biologischen Wirkung der erfindungsgemäßen Zusammensetzung ist die Untersuchung der durch Allergie ausgelösten Asthmasymptome bei den Mäusen. Abb. 4 zeigt, dass die Empfindlichkeit der Atemwege bei den asthmatischen Mäusen durch die Behandlung mit dem erfindungsgemäßen Extrakt auf das Niveau von unbehandelten Mäusen ohne Asthma abgesenkt werden kann. Um auszuschließen, dass der biologische Effekt der erfindungsgemäßen Zusammensetzung nicht durch LPS bedingt ist, wurde reines LPS in einer Konzentration, wie sie in den Stallstaubextrakten gemessen wurden, als Kontrolle eingesetzt. LPS kann die Asthmasymptome der Mäuse nicht reduzieren. Man muss also davon ausgehen, dass andere Substanzen im Staub für den beobachteten biologischen Effekt verantwortlich sind. CHCl₃/MeOH ist die klassische Extraktionsmethode, mit der nach Phasentrennung Lipide in die Chloroformphase und andere Moleküle mit schwächeren lipophilen Eigenschaften in die Methanolphase extrahiert werden. Der Methanol-Extrakt (hier CHCl₃/MeOH genannt) hat keinen präventiven Einfluss auf die Asthmasymptome der Mäuse. Der in H₂Oₖₐₗₜ extrahierte Stallstaub bewirkt eine partielle Reduktion der Symptome, die aber nicht so effektiv ist wie die Wirkung der erfindungsgemäßen Zusammensetzung.

***Entzündung in den Bronchien** -* Bei Mäusen mit Asthma reflektieren eosinophile Zellen in den Bronchien den Zustand einer asthmatischen Entzündung. Abb. 5 belegt, dass durch die Behandlung der Mäuse mit der erfindungsgemäßen Zusammensetzung die Zahl der eosinophilen Zellen, die kranken Mäusen stark erhöht sind und im Normalfall fehlen, stark absinkt und nur noch geringfügig zu finden sind. Wie bei den asthmatischen Symptomen findet man bei den mit Puffer und LPS behandelten Mäusen keine Veränderung, CHCl₃/MeOH hat nur einen geringen Einfluss, der H₂Oₖₐₗₜ Extrakt erreicht nicht ganz die Wirkung der erfindungsgemäßen Zusammensetzung.

**Allergische Sensibilisierung -** Die Asthmasymptome werden in den Mäusen durch Sensibilisierung mit einem Allergen ausgelöst. Deshalb wurde untersucht, ob die bei Sensibilisierung ansteigenden IgE-Antikörper gegen das Allergen durch die erfindungsgemäßen Zusammensetzung beeinflusst werden. Die Tabelle dokumentiert, dass die gegen Ovalbumin gerichteten lgE Antikörper lediglich bei Anwendung der erfindungsgemäßen Zusammensetzung im Vergleich zu den unbehandelten Mäusen statistisch signifikant abfallen, bei den anderen Kontrollen aber nicht oder zumindest nicht signifikant. Der Anstieg der lgG1 Antikörper bei Ovabuminsensibilisierung korreliert zwar nicht mit den Asthmasymptomen, reflektiert aber eine Reaktion der T-Lymphozyten vom Typ Helfer-2 (Th2), deren Erhöhung bei Allergien zu beobachten sind. Hier zeigt die Tabelle, dass sowohl die erfindungsgemäße Zusammensetzung als auch die beiden anderen Staubextrakte die Th2 Antwort des Immunsystems reduzieren können, nicht jedoch das LPS. Die Th2 Antwort des Immunsystems ist also kein präziser Marker für den besonderen biologischen Effekt der erfindungsgemäßen Zusammensetzung. Außerdem scheinen die wirksamen Substanzen, die in der erfindungsgemäßen Zusammensetzung wirken, in den anderen Extrakten außer in der LPS-Lösung partiell ebenfalls enthalten zu sein.

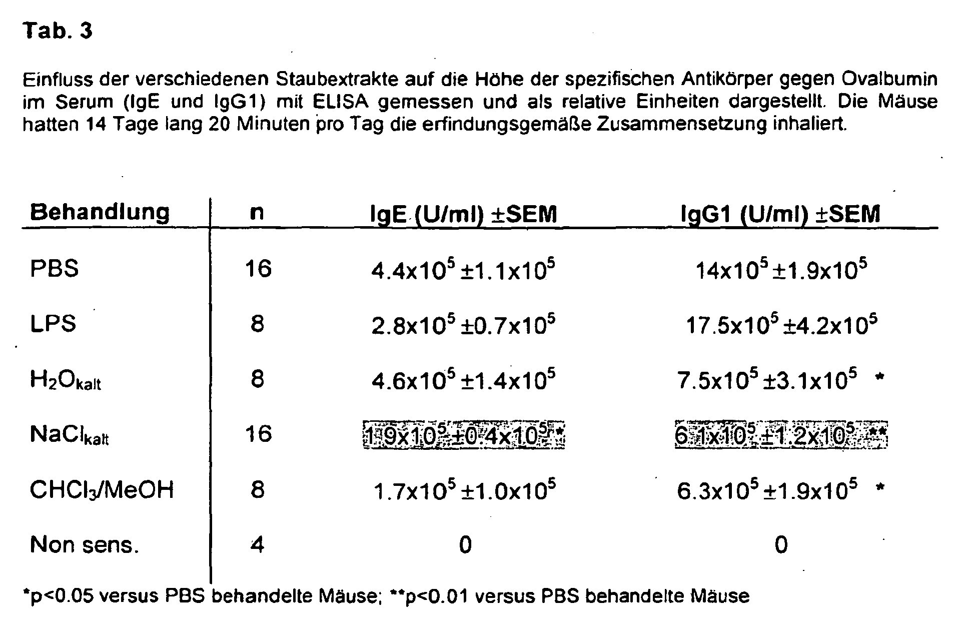

## Patentansprüche

1. Verfahren zur Herstellung eines als Medikament zu verwendenden anti-allergenen Extraktes, **dadurch gekennzeichnet, dass** der Extrakt in folgenden Schritten hergestellt wird:
a) Sammeln von trockenem Stallstaub,
b) gegebenenfalls Homogenisieren des Staubs,
c) Suspendieren des ggf. homogenisierten Staubs in Wasser oder wässriger Puffer-Lösung, enthaltend ein- oder zweiwertige Salze, ausgewählt aus NaCl, Na₂SO₄, KCl, LiCl, MgCl₂ und CaCl₂, bevorzugt 0,9% NaCl-Lösung bei Umgebungstemperatur oder darunter,
d) gegebenenfalls Aufschließen der Staubbestandteile,
e) Sedimentieren der festen Teile der Suspension und Abnahme des wässrigen Überstandes, wobei der Überstand nach der Sedimentation
f) gegebenenfalls Dialyse des wässrigen Überstandes, den Extrakt darstellt,
wobei während des gesamten Herstellungsverfahrens keine Wärmezufuhr erfolgt.

2. Verfahren nach Anspruch 1, **dadurch gekennzeichnet, dass** der Extrakt aus Stallstaub von Bauernhöfen hergestellt wird.

3. Verfahren nach einem der Ansprüche 1 oder 2, **dadurch gekennzeichnet, dass** der Extrakt in folgenden Schritten hergestellt wird:
a) Sammeln von trockenem Stallstaub,
b) Homogenisieren des Staubs,
c) Suspendieren des homogenisierten Staubs in wässriger 0,9% NaCl-Lösung bei Umgebungstemperatur oder darunter,
d) gegebenenfalls Aufschließen der Staubbestandteile,
e) Sedimentieren der festen Teile der Suspension,
f) Abnahme des Überstandes und Dialyse des wässrigen Überstandes.

4. Extrakt, hergestellt nach einem Verfahren gemäß einem der Ansprüche 1 bis 3.

5. Extrakt nach Anspruch 4, **dadurch gekennzeichnet, dass** er Saponine und/oder Lipoglycane enthält.

6. Medikament in Form eines Extraktes gemäß Anspruch 4 oder 5.

7. Extrakt gemäß einem der Ansprüche 4 bis 6 als Medikament zur Vorbeugung oder Behandlung von allergenen Erkrankungen, insbesondere von Asthma bronchiale, Heuschnupfen, atopischer Dermatitis, Nahrungsmittelallergien, Urtikaria oder Kontaktallergien.

## Claims

1. A process for the preparation of an antiallergenic extract usable as medicament, **characterized in that** the extract is prepared in the following steps:
a) collecting of dry barn dust,
b) optionally homogenizing the dust,
c) suspending the optionally homogenized dust in water or aqueous buffer solution comprising monovalent or divalent salts, selected from NaCl, Na₂SO₄, KCl, LiCl, MgCl₂ and CaCl₂, preferably 0.9% strength NaCl solution at ambient temperature or below,
d) optionally disrupting the dust components,
e) sedimenting the solid constituents of the suspension, and
f) optionally dialysis of the aqueous supernatant, whereby the supernatant constitutes the extract after sedimentation,
whereby no heat is added during the entire preparation process.

2. The process as claimed in claim 1, **characterized in that** the extract is prepared from barn dust from farms.

3. The process as claimed in claims 1 or 2, **characterized in that** the extract is prepared in the following steps:
a) collecting of dry barn dust,
b) homogenizing the dust,
c) suspending the homogenized dust in aqueous 0.9% strength NaCl solution at ambient temperature or below,
d) optionally disrupting the dust components,
e) sedimenting the solid constituents of the suspension,
f) removal of the supernatant and dialysis of the aqueous supernatant.

4. Extract prepared according to a process as claimed in any of claims 1 to 3.

5. Extract as claimed in claim 4, **characterized in that** it contains saponins and/or lipoglycanes.

6. Medicament in the form of an extract as claimed in claim 4 or 5.

7. Extract as claimed in any of claims 4 to 6 as medicament for the prevention or treatment of allergenic diseases, especially bronchial asthma, hayfever, atopic dermatitis, food allergies, urticaria or contact allergies.

## Revendications

1. Procédé de production d'un extrait anti-allergène à utiliser comme médicament, **caractérisé en ce que** l'extrait est produit dans les étapes suivantes :
a) recueil de poussière d'étable sèche,
b) éventuellement, homogénéisation de la poussière,
c) suspension de la poussière éventuellement homogénéisée dans de l'eau ou une solution de tampon aqueuse, contenant des sels mono- ou divalents choisis parmi les NaCl, Na₂SO₄, KCl, LiCl, MgCl₂ et CaCl₂, de préférence, une solution de NaCl à 0,9 % à température ambiante ou à une température inférieure,
d) éventuellement, décomposition des composants de la poussière,
e) sédimentation des parties solides de la suspension, et réduction du surnageant aqueux, le surnageant représentant l'extrait après sédimentation,
f) éventuellement, dialyse du surnageant aqueux,
sachant que, pendant le procédé de production complet, il n'y a pas d'apport de chaleur.

2. Procédé selon la revendication 1, **caractérisé en ce que** l'extrait est produit à partir de poussière d'étable de fermes.

3. Procédé selon l'une des revendications 1 ou 2, **caractérisé en ce que** l'extrait est produit dans les étapes suivantes :
a) recueil de poussière d'étable sèche,
b) homogénéisation de la poussière,
c) suspension de la poussière homogénéisée dans une solution aqueuse de NaCl à 0,9 % à température ambiante ou à une température inférieure,
d) éventuellement, décomposition des composants de la poussière,
e) sédimentation des parties solides de la suspension,
f) réduction du résidu et dialyse du surnageant aqueux.

4. Extrait produit d'après un procédé selon l'une des revendications 1 à 3.

5. Extrait selon la revendication 4, **caractérisé en ce qu'**il contient de la saponine et/ou du lipoglycane.

6. Médicament sous forme d'extrait selon la revendication 4 ou 5.

7. Extrait selon l'une des revendications 4 à 6 servant de médicament en prévention ou en traitement de maladies allergènes, en particulier, de l'asthme bronchique, du rhume des foins, de la dermatite atopique, d'allergies alimentaires, de l'urticaire ou d'allergies de contact.
